# FASCICULE DE BREVET EUROPEEN

(11) **EP 2 067 392 B1**
(45) Date de publication et mention de la délivrance du brevet: **30.01.2013**
(21) Numéro de dépôt: 07820566.3
(22) Date de dépôt: 25.09.2007
(51) Int. Cl.: H05K 9/00, A61N 1/16

(54) **DISPOSITIF DE PROTECTION CONTRE DES EFFETS D'UNE ONDE DE TORSION EMISE PAR UN APPAREIL ELECTRONIQUE**
EINRICHTUNG ZUM SCHUTZ VOR EFFEKTEN EINER VON EINER ELEKTRONISCHEN VORRICHTUNG EMITTIERTEN TORSIONSWELLE
DEVICE FOR PROTECTING AGAINST EFFECTS OF A TORSION WAVE EMITTED BY AN ELECTRONIC APPARATUS

(30) Priorité: 25.09.2006 FR 0608394
(43) Date de publication de la demande: 10.06.2009
(73) Titulaire: Vital Concept, 22600 Loudeac (FR)
(72) Inventeur: PAVLENKO, Anatoly, Kiev, 03115 (UA); ETIENNE, Patrice, 22603 Loudeac (FR); RUSANOV, Alexandre, 29217 Le Conquet (FR)
(74) Mandataire: Bioret, Ludovic
(86) Numéro de dépôt international: PCT/EP2007/060171
(87) Numéro de publication internationale: WO 2008/037719

(56) Documents cités:
- WO-A-02/40859
- WO-A-02/40860
- WO-A-2004/088789
- WO-A1-02/18007
- FR-A1- 2 692 155
- RU-C1- 2 127 614
- US-A1- 2003 011 530

## Description

### 1. DOMAINE DE L'INVENTION

La présente invention se situe principalement dans le domaine de la protection des personnes contre les effets des champs de torsion. Un domaine connexe est celui de la prévention des risques liés à l'usage d'appareils produisant leur propre champ de torsion.

Plus précisément, l'invention concerne un dispositif de protection contre les effets du champ de torsion accompagnant le champ électromagnétique généré par des appareils électroniques.

L'invention trouve de nombreuses applications, telles que par exemple la protection des usagers de téléphone cellulaire ou d'assistant personnel (PdA) ou encore des utilisateurs d'ordinateurs ou de téléviseurs.

Plus généralement, elle peut s'appliquer dans tous les cas où un appareil comprend des composants électroniques.

### 2. ART ANTÉRIEUR

### 2.1. Contexte

À l'échelle particulaire, des états énergétiques d'excitation particuliers des électrons, des protons ou des neutrons peuvent être la source d'un champ de torsion élémentaire. Birrel et al. ont ainsi mis en évidence la composante du champ de torsion élémentaire correspondant au spin classique ½ (Birrel N.D., Davis P.C.V. Quantum fields in curves space. Cambridge University Press. Cambridge, London, New York, New Roshelle, Melbourn, Sydney, 1982, 386 p.). Plus récemment, dans le cadre d'un programme de recherche sur lés influences bioénergétiques à distance, V.V. Kvartalnov et N.F. Perevoztchikov ont isolé, en sus du rayonnement électromagnétique, un rayonnement de torsion, encore appelé rayonnement spinoriel, ou ondes de torsion, au sein du faisceau d'un laser quantique ("Découverte d'un composant non physique de rayonnement OKG", Revue du fond de parapsychologie L. L. Vassiliev, 1999, N°2 (28), art. 64-67).

Des champs de torsion peuvent également être présents à une échelle macroscopique. De tels champs résultent de l'ordonnancement collaboratif des spins de particules nucléaires ou atomiques d'un corps physique (Akimov A.E., Tarassenko V.Y. : Informations des Grandes Ecoles, série Physique, 1992, T.35, N°3, p.13). En d'autres termes, puisque tout corps organique ou minéral possède son propre arrangement stéréochimique, qui se caractérise par la position des atomes au sein des molécules et par l'orientation respective des spins des atomes et des particules nucléaires associées, la superposition des champs de torsion élémentaires de chacune des particules contribue à créer un champ de torsion macroscopique dont l'intensité et la structure dépendent du moment cinétique de spin qui lui est associé.

Par exemple, il a été mis en évidence par D.J. Carlson ("Dynamic Nuclear Orientation". New-York - London - Sydney, John Wieley & Sans, 1963, 485 p.) qu'un corps dont la structure atomique est à noyaux orientés (par exemple un corps ferromagnétique aimanté) et, plus généralement, un corps comprenant des particules dont les spins atomiques et nucléaires sont parallèles et de même direction, génère un champ de torsion.

Ainsi, quelle que soit sa nature ou sa composition, tout corps organique ou minéral peut générer son propre champ de torsion. En particulier, il est connu que les corps dont la forme présente des singularités géométriques, comme par exemple les pyramides, les cônes, les tridents, les cylindres et les triangles plats sont des sources naturelles actives de champs de torsion (A.R. Pavlenko : « Ordinateur, TV et santé ». Nikolaïev. « Quid », 2003, p. 26). De tels champs de torsion sont aussi appelés champs de torsion statiques de forme.

Une extension du modèle quantique de là « Mer de Dirac » a été proposée par A. E. Akimov et al pour expliquer l'existence de champ de torsion au sein du Vide Physique (« Modèle d'états de polarisation du Vide Physique », Information des grandes écoles - 1992.-N°3, Physique - p.13-23). Dans ce modèle, le Vide Physique est interprété comme un système de paquets d'ondes électron-positron, ce qui permet d'expliquer l'origine des champs de torsion à partir de la polarisation torsionnelle des spins de particules. Ce modèle permet également de révéler l'origine du paradoxe de dissymétrie des champs de torsion, par opposition à la symétrie des champs électromagnétiques ou gravitaires qui est imposée par la théorie des champs. Ainsi les lignes de champ d'un champ de torsion émis par un corps physique s'organisent suivant sensiblement deux cônes dirigés dans des directions opposées. A chaque premier demi-espace auquel est associé un premier des deux cônes correspond un premier champ de torsion dont les propriétés ne peuvent pas être déduites par symétrie du second champ de torsion présent dans le second demi-espace complémentaire. Ces premier et second champs peuvent être classifiés suivant deux types de champs classiquement appelés champ de torsion gauche et champ de torsion droit, les champs de torsion gauche étant notamment caractérisés par une vorticité anti-horaire. Leurs intensités respectives dépendent notamment de la forme du corps physique.

L'état des spins des particules ou des molécules d'un corps physique peut être influencé par un champ de torsion provenant d'une source externe et peut transiter vers un nouvel état d'équilibre métastable, ce qui entraîne une modification de la structure du champ de torsion propre du corps. Il en résulte que la structure de tout corps physique, et, en particulier, des corps organiques tels que des bactéries ou de cellules biologiques, peut être transformée en soumettant ses particules à un champ de torsion externe.

Parmi les sources de champs de torsion, on connaît également les sources de champs électrostatiques ou électromagnétiques. En effet, du fait qu'un champ électrostatique(/électromagnétique) provoque une polarisation électronique et atomique(/photonique) et donc une modification de l'état de spin des porteurs de charge, les champs électrostatiques(/électromagnétiques) sont donc aussi toujours des sources de champ de torsion.

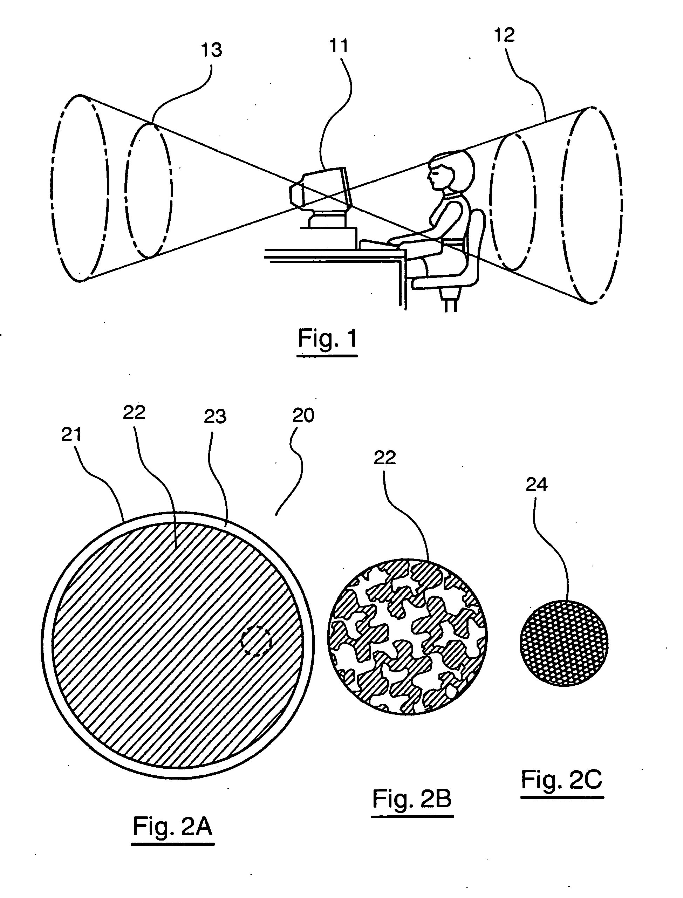

Ainsi la plupart des appareils électroniques, parmi lesquels les téléphones cellulaires, les écrans d'ordinateur ou les télévisions, les photocopieurs..., dont le fonctionnement repose sur l'utilisation d'ondes électromagnétiques, sont par conséquent des sources de champs de torsion gauches et droits. La figure 1 illustre la structure en forme de cône des lignes d'un champ de torsion gauche 12, et d'un champ de torsion droit 13 émis par un écran d'ordinateur 11. Il a en effet été constaté que d'une manière générale pour les appareils électroniques diposant d'un écran tels que, par exemple, les ordinateurs, les téléphones cellulaires ou les téléviseurs, des ondes de torsion gauche et droite sont émises dans un plan orthogonal à l'écran, l'onde de torsion gauche(/droite) rayonnant en direction de la face avant(/arrière) de l'écran.

Alors qu'on a longtemps pensé que les ondes électromagnétiques émises par les appareils électroniques, et en particulier par les appareils électroniques de petite taille tels que le téléphone cellulaire, l'ordinateur de poche,... était probablement à l'origine des troubles de santé constatés par des médecins chez les utilisateurs de tels appareils, ce phénomène médical, se traduisant notamment par une modification du taux de cholestérol dans le sang ou par l'apparition de troubles nerveux ou cutané, ayant été qualifié d'hypersensibilité électromagnétique, des conclusions d'un récent rapport d'experts scientifiques commandé par l'Union Européenne semblent confirmer le contraire (« Final Report : Risk evaluation of potential environnemental Hazard from Low Frequency Electromagnetic Field Exposure using Sensitive in vitro Methods » - contrat QLK4-CT-199-01574, réalisé entre le 1er février 2000 et le 31 mai 2004).

Un programme de recherche mené à la Faculté de Biologie de l'Université d'Etat de Moscou a en outre montré que le phénomène d'hypersensibilité électromagnétique conservait la même intensité que les appareils soient en marche ou que l'on ait retiré le bloc d'alimentation. L'équipe ayant mené ces recherches a de plus identifié que la cause probable du phénomène d'hypersensibilité électromagnétique était l'utilisation de circuits intégrés multicouches de dernière génération, possédant des structures matricielles très compactes.

En revanche, des expériences menées dans plusieurs centres scientifiques russes, dont la faculté de Biologie de l'Université d'Etat de Moscou, ont montré que l'exposition à un champ de torsion gauche généré par des appareils électroniques tels qu'un téléphone cellulaire, un ordinateur,..., peut avoir un effet négatif sur le corps humain (V.A. Nekrassov, « la vie sur terre - série : les secrets de la nature et les découvertes du siècle » , Edition Triada, 2004). En particulier, les effets négatifs du champ de torsion gauche peuvent se manifester par une dégradation du système de défense immunitaire et une plus grande vulnérabilité aux maladies d'origines infectieuses. Au contraire, l'exposition à un champ de torsion droit semble avoir un effet positif sur la santé.

Les constats précédents laissent donc apparaître que l'origine du phénomène d'hypersensibilité électromagnétique est de nature spinorielle, et donc liée à l'exposition aux ondes de torsion gauche créées par les circuits intégrés multicouches de dernière génération.

### 2.2. Solutions de l'art antérieur

Divers dispositifs de protection, de type à écran ou de type à compensation de champ, ont été proposés pour atténuer l'effet du rayonnement électromagnétique.

Une technique connue d'écran de protection contre des champs électromagnétiques a été commercialisée par la société Zuccary sous la dénomination « Saffety Butterfly ». L'écran selon cette technique, destiné à être fixé sur un téléphone cellulaire, encore appelé téléphone portable, se compose de quatre demi-sphères principales en matériau amorphe non magnétique et de deux demi-sphères de diamètre inférieur. Chacune de ces demi sphères contient 12 pour-cent d'une solution d'hydrate de carbone et est reliée à sa base à un support comprenant une feuille de cuivre et une feuille d'aluminium.

Un inconvénient de cette technique d'écran est que l'écran tout en réfléchissant les ondes électromagnétiques dans une direction nuit à l'émission et la réception des communications transmises par ondes électromagnétiques, et donc perturbe le fonctionnement du téléphone portable.

On connaît aussi une technique mise au point en Russie pour protéger les personnes des ondes électromagnétiques par génération d'un champ de compensation. L'appareil selon cette technique, dénommé DAR, se présente sous la forme d'un générateur d'ondes électromagnétiques annihilant le rayonnement externe et opérant dans la gamme des très hautes fréquences comprises entre 30 et 300 GigaHertz.

Un premier inconvénient de cette technique par génération d'un champ de compensation, partagé avec la technique d'écran décrite ci-dessus, est que la mise en oeuvre d'un tel dispositif perturbe le fonctionnement des appareils électroniques.

En outre, un deuxième inconvénient de cette technique, est que son coût de mise en oeuvre reste élevé.

Ainsi dans le cadre du développement de techniques permettant de protéger les utilisateurs d'appareils électroniques, et notamment les usagers de téléphones portables, d'ordinateurs ou de télévision, des effets du champ de torsion gauche généré par de tels appareils, on a recherché des solutions techniques dont la mise en oeuvre ne perturbe pas le champ électromagnétique fonctionnel de l'appareil. Il va donc de soi que les techniques susmentionnées, par écran ou mettant en oeuvre un générateur d'ondes électromagnétiques, ne conviennent pas, et qu'il a fallu rechercher des solutions reposant sur des principes d'action différents.

Une première technique connue de l'art antérieur décrite dans le brevet américain US 6,548,752 concerne un appareil de protection composé de deux sphères concentriques en matériau vitreux non magnétique, telles que la sphère extérieure contient une solution d'hydrate de carbone et qu'une couche d'une solution de sels rares dans laquelle peut se déplacer un élément magnétique, permettant de générer un champ de torsion droit, occupe l'espace entre les deux sphères. L'élément magnétique s'oriente librement par rapport aux pôles magnétiques terrestres ce qui permet d'amplifier son champ de torsion droit.

Une deuxième technique de protection contre le rayonnement de torsion des ordinateurs et des téléviseurs, proposée à la vente par la société suisse S.E.I.G et mise en oeuvre par l'intermédiaire de deux mini-sphères en plastique aux centres desquelles sont placées des solutions de métaux rares, est proche de cette première technique connue.

Une troisième technique connue de l'art antérieur, décrite dans le document de brevet français FR 2 692 155, est mise en oeuvre au moyen d'un dispositif comprenant deux tubes accolés remplis de liquide dont les extrémités sont recouvertes de sphères en acier. Ce dispositif est destiné à être fixé sur le panneau gauche de l'écran d'un ordinateur.

Une quatrième technique connue de l'art antérieur, décrite dans le document de brevet ukrainien n°29839, publié le 15.10.2001, concerne un appareil de protection des utilisateurs de PC et de téléviseurs contre l'effet néfaste dés ondes de torsion. L'appareil renferme un élément neutralisateur, fait d'au moins deux cylindres concentriques placés l'un dans l'autre, l'espace entre les cylindres étant comblé par du polymère. Ces cylindres sont de même hauteur, le diamètre du plus petit de ces cylindres correspondant à plus de la moitié de sa hauteur. D'autre part, ces cylindres sont fixés sur un support, qui possède sur son côté externe des sillons en forme d'une spirale déroulée dans le sens anti-horaire. L'appareil se présente comme un générateur de champ de torsion statique de forme droit. Le champ sommaire de torsion statique de forme droit de l'appareil agit en écartant le champ de torsion gauche incident généré par le tube cathodique de l'écran.

Le principe de protection de ces quatre techniques connues de l'art antérieur décrites ci-dessus repose ainsi sur la génération d'un champ de torsion statique de forme droit qui interagit avec le champ de torsion gauche néfaste produit par des appareils électroniques ou propre à l'environnement naturel. En effet selon le principe d'interaction mutuelle des ondes de torsion gauche et droite, les champs de torsion gauche et droite tendent à s'écarter.

Une cinquième technique de l'art antérieur décrite dans le document de brevet de la fédération de Russie N2127614 concerne une grille de diffraction des ondes de torsion gauche destinée à la protection des personnes contre les effets des zones terrestres émettant des ondes de torsion pathogènes. Cette grille de diffraction est réalisée à partir d'un tissu métallique comprenant une maille régulière d'anneaux entrecroisés de diamètre 50 à 300 millimètres dont la section est ronde ou carrée et dont l'épaisseur varie entre 0,5 et 10 millimètres. Les métaux utilisés pour réaliser cette grille de diffraction sont le cuivre, l'aluminium, l'or ou leurs alliages.

Le principe de cette technique consiste à diffracter et donc à dévier les ondes de torsion en faisant interférer les ondes de torsion dans les mailles de la grille.

Une sixième technique de l'art anterieur est connue et décrite dans le demande de brevet internationale WO 02/18007 concerne un dispositif pour protéger les personnes contre les champs de torsion avant une polarisation lévogyre (gauche) de l'espace, qui comprend un l'élément de compensation enrobé ayant la forme de deux socles coaxiaux arrangés sur une base commune.

### 2.3. Inconvénients des solutions de l'art antérieur.

Un inconvénient de la première, ou de la deuxième, ou de la troisième, ou de la quatrième technique connue de l'art antérieur est qu'une source de champ de torsion droit génère inévitablement un champ de torsion gauche propre dont l'intensité varie en fonction de l'environnement immédiat de l'appareil, ce qui peut réduire la protection recherchée avec une telle technique.

Un premier inconvénient de la cinquième technique connue de l'art antérieur, identifié par les inventeurs de la présente demande de brevet, est que la taille des mailles de la grille est trop importante pour permettre à des ondes de torsion gauche émises dans la gamme de fréquence des appareils électroniques d'interférer efficacement, ce qui réduit l'angle de déviation des ondes de torsion gauche.

Un deuxième inconvénient de la cinquième technique connue de l'art antérieur, est que du fait de la régularité de la maille, la bande passante de la grille utilisée selon cette technique est limitée, et, en d'autres termes, il s'agit d'une grille de diffraction sélective.

Un autre inconvénient de cette cinquième technique connue est que les matériaux paramagnétiques utilisés par cette technique peuvent interagir avec le champ électromagnétique des appareils électroniques.

Encore un autre inconvénient de cette cinquième technique connue est sa taille ainsi que son prix de revient élevé du fait des matériaux préconisés.

### 3. OBJECTIFS DE L'INVENTION

L'invention, dans au moins un mode de réalisation, a notamment pour objectif de pallier ces différents inconvénients de l'état de la technique.

Plus précisément, l'un des objectifs de la présente invention, dans au moins un mode de réalisation, est de fournir une technique de protection de la santé des personnes en présence d'appareils électroniques.

Un autre objectif de l'invention est de de fournir une technique pouvant être mise en oeuvre, sans modifier le dispositif, sur une large bande passante correspondant à la gamme de fréquence des appareils électroniques usuels.

Un autre objectif de l'invention est de fournir une telle technique qui ne perturbe pas les ondes électromagnétiques.

Un autre objectif de l'invention est de fournir une telle technique qui soit simple à mettre en oeuvre et compacte.

Un autre objectif de l'invention est la fourniture d'une telle technique qui soit robuste.

L'invention à encore pour objectif de fournir une telle technique qui soit simple à produire et qui ne soit pas onéreuse.

### 4. EXPOSÉ DE L'INVENTION

Ces objectifs, ainsi que d'autres qui apparaîtront par la suite, sont atteints à l'aide d'un dispositif de protection contre des effets d'un champ de torsion émis selon une direction par un appareil incluant des composants électroniques opérant dans une gamme de fréquence comprise entre un Mégahertz et trois Gigahertz comprenant un élément de diffraction dudit champ de torsion.

Selon l'invention, ledit élément de diffraction comprend une pluralité de passages dont l'ouverture est de largeur inférieure ou égale à un millimètre dans au moins une direction, et au moins deux desdits passages présentent des pourtours de formes différentes.

Ainsi, dans au moins un mode de réalisation, l'invention repose sur une approche tout à fait nouvelle et inventive de la protection envers le rayonnement de torsion émis par un appareil électronique consistant à mettre en oeuvre un élément de diffraction des ondes de torsion peu sélectif, dont les passages sont d'ouverture très petite devant la longueur d'onde des ondes de torsion afin de dévier le champ de torsion incident, et non identiques afin d'élargir sa bande passante. On note que les inventeurs ont évalué qu'une ouverture d'un millimètre permet de diffracter correctement un champ de torsion incident généré par des circuits intégrés d'un appareil électronique cadencés à trois GigaHertz.

Dans un mode de réalisation avantageux de l'invention, ledit élément de diffraction comprend une grille, et la distribution desdits pourtours sur ladite grille est fractale.

Ainsi, on dispose, d'une part, d'une grille de large bande passante du fait de la répartition fractale des pourtours sur la grille, et, d'autre part, d'une source de champ de torsion droit amplifiée du fait qu'une disposition fractale coïncide avec le schéma des relations réciproques spatiales stables (emballage compact de fitons non perturbés) ou, en d'autres termes, du fait que le schéma structurel de l'arrangement du Vide Physique non perturbé (situation de résonance), pour lequel la génération de champ de torsion droit est maximale, est de type fractal.

Selon une caractéristique avantageuse, au moins un desdits pourtours est de forme sensiblement circulaire et/ou elliptique.

Selon une autre caractéristique avantageuse, ladite grille comprend un maillage d'éléments courbes et/ou brisées entrecroisés.

Ainsi, on obtient une grille de diffraction bi- ou multi-couches permettant d'amplifier l'effet de diffraction.

Préférentiellement, ladite grille présente une densité supérieure et/ou égale à 19.

Ainsi, on accroît le saut de densité de matière à l'interface avec le Vide Physique de façon à favoriser l'effet de diffraction.

Dans un autre mode de réalisation avantageux, ledit élément de diffraction comprend un ensemble de particules en vrac dont les intervalles séparateurs définissent ladite pluralité de passages.

Ainsi, on obtient simplement une grille de diffraction peu sélective.

Selon une caractéristique avantageuse, au moins une desdites particules comprend au moins un bulbe.

Ainsi, on favorise la génération par l'élément de diffraction d'un champ de torsion droit destiné à interagir avec le champ de torsion gauche incident, puisqu'il est établi de façon scientifique que les corpus prenant la forme d'un bulbe, et plus particulièrement, selon des données issues de la microscopie électronique (P. Harris : « nanotubes de carbone et structures apparentées. Les nouveaux matériaux du XXI siècle ». Technosphère, p.18), les bulbes réalisés par deux sphères concentriques, peuvent générer de tels champs.

De façon avantageuse, ledit bulbe comprend au moins une saillie prolongée par une pointe de façon à générer en outre un champ modifiant ladite direction du champ de torsion.

Ainsi, comme expliqué auparavant, une singularité géométrique étant source d'un champ de torsion statique de forme droit, l'onde de torsion droite émise par l'élément de diffraction est amplifiée.

Selon une caractéristique préférentielle, lesdites particules sont réalisées dans une des matières appartenant au groupe comprenant :
- carbone ;
- silice ;
- métal ;
- carbonate de calcium.

Dans un mode de réalisation particulier de l'invention ledit élément de diffraction est enfermé dans une enveloppe fermée en matériau amorphe.

Ainsi, on protège le dispositif des chocs ou des agressions chimiques sans perturber l'action de l'élément de diffraction.

Dans un autre mode de réalisation particulier de l'invention, la surface externe de ladite enveloppe fermée présente une surface externe définie par une pluralité de couches superposées dans la direction dudit champ de torsion et formant des degrés successifs dont la largeur est supérieure à la hauteur.

Ainsi, on réalise un empilement d'éléments de diffraction distincts de façon à amplifier l'effet de diffraction.

### 5. LISTE DES FIGURES

D'autres caractéristiques et avantages de modes de réalisation de l'invention apparaîtront à la lecture de la description suivante de deux modes de réalisation particuliers de l'invention, donnés à titre d'exemples indicatifs et non limitatifs (tous les modes de réalisation de l'invention ne sont pas limités aux caractéristiques et avantages de ces modes de réalisation particuliers), et des dessins annexés, dans lesquels :
- la figure 1 illustre la distribution spatiale des cônes symétriques du rayonnement de torsion d'un écran d'ordinateur (associés à un champ de torsion gauche et un champ de torsion droit) ;
- la figure 2A présente un dispositif de protection contre les effets d'un champ de torsion émis par un téléphone portable selon un mode de réalisation particulier de l'invention ;
- la figure 2B est une vue de détail de la grille fractale du dispositif de la figure 2A;
- la figure 2C illustre une vue de détail d'une variante du mode de réalisation présenté à la figure 2A pour laquelle la structure fractale des contours des passages est sensiblement circulaire ;
- la figure 3A présente un dispositif de protection contre les effets d'un champ de torsion émis par un téléphone portable selon un autre mode de réalisation particulier de l'invention ;
- la figure 3B illustre la déviation du champ de torsion gauche d'un téléviseur produite par la mise en oeuvre du dispositif de protection présenté à la figure 3A.

On notera que dans sur toutes les figures du présent document et dans la description détaillée suivante de la présente invention, des éléments identiques sont désignés par une même référence numérique.

### 6. DESCRIPTION DÉTAILLÉE

### 6.1. Rappel du principe de l'invention

Comme ceci a été précédemment indiqué, le principe général de l'invention repose sur la mise en oeuvre d'un élément de diffraction des ondes de torsion dont les passages possèdent une ouverture très petite devant la longueur d'onde des ondes de torsion et sont, au moins pour certains d'entre-eux, dissemblables, de façon à dévier sensiblement le champ de torsion incident généré par tout type d'appareil électronique fonctionnant dans la gamme de fréquence comprise entre 1Mhz et 3GigaHertz, c'est-à-dire suivant un angle de déviation supérieur à 90°.

Cette approche permet ainsi de protéger efficacement les utilisateurs de téléphones portables, de téléviseurs, ou d'ordinateurs, ... contre le rayonnement de torsion gauche émis par ces appareils.

En outre, les caractéristiques du dispositif selon l'invention permettent de concevoir un dispositif compact, facilement intégrable dans un appareil électronique.

### 6.2. Premier mode de réalisation particulier de l'invention

On présente en relation avec la figure 2A un mode de réalisation d'un dispositif selon l'invention de protection contre les effets des ondes de torsion émises par un téléphone portable de type bi-bande.

Le dispositif de protection 20 comprend un élément de diffraction 21 incluant une grille de diffraction 22 circulaire, de diamètre seize millimètres, enveloppée dans une gaine plastique 23. Cette gaine plastique 23 permet d'éviter que la grille 22 ne se détériore du fait de chocs et/ou d'un dépôt de particules ou de fluides polluants et/ou agressifs.

Dans une variante de ce mode de réalisation de l'invention, il peut être également envisagé d'empiler plusieurs grilles de diffraction pour former l'élément de diffraction.

La figure 2B précise le détail de la géométrie de la grille de diffraction. Le schéma des passages de la grille a été obtenu à partir de l'indentation du motif d'arborescence d'une courbe de Von Koch généralisée à 64 segments. On rappelle qu'une grille de diffraction fractale agit comme un résonateur de contours coïncidant avec le schéma structurel des relations réciproques spatiales inhérentes au Vide Physique non perturbé. En d'autres termes en fixant l'élément de diffraction sur la partie interne de la coque supérieure du téléphone portable, on rétablit les conditions d'une résonance dynamique optimale du spectre des modifications biologiques au voisinage du téléphone.

Bien entendu la structure géométrique fractale peut être obtenue à partir de toute autre figure fractale, sans sortir du cadre de l'invention. À titre d'illustration, la figure 2C présente une vue de détail d'une grille 24 pour laquelle la structure fractale des contours des passages est sensiblement circulaire.

Les passages de la grille, de contours différents, sont d'une dimension moyenne de 50 micro-mètres pour diffracter de façon efficace les ondes de torsion autour des fréquences 900 et 1800 MégaHertz imposées par la norme européenne GSM (littéralement en anglais « Global System for Mobile communication").

### 6.3. Deuxième mode de réalisation particulier de l'invention.

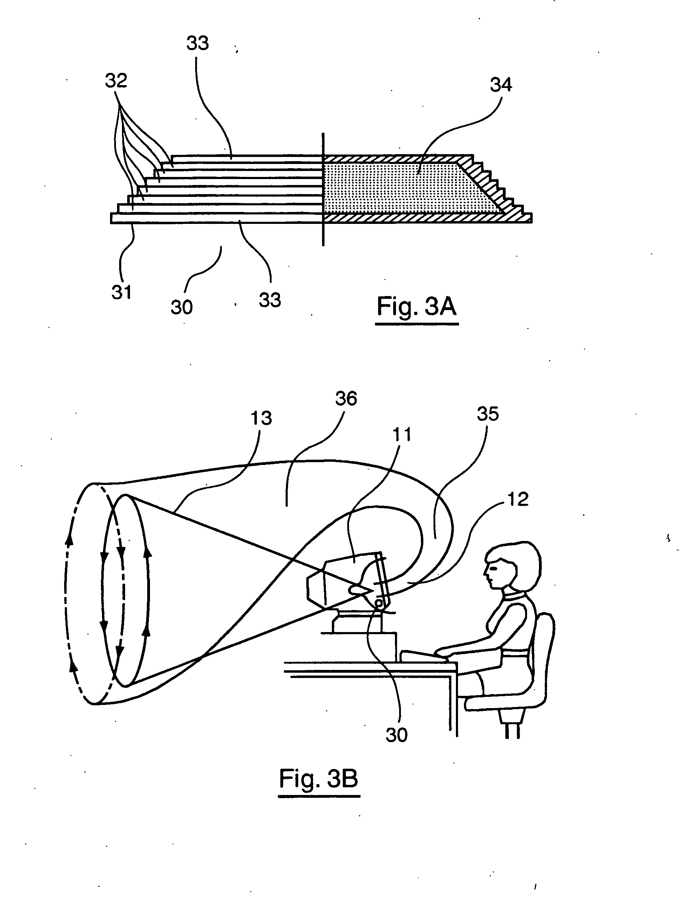

On présente, en relation avec la figure 3A, un deuxième mode de réalisation d'un dispositif de protection 30 selon l'invention.

Dans ce mode de réalisation, le dispositif 30 est plaqué, pour des raisons pratiques, contre la face du panneau à gauche de l'écran. Il est toutefois clair que ce dispositif peut être placé dans toute position en avant du plan du circuit imprimé du tube cathodique sur lequel sont fixés les composants électroniques, et notamment les circuits intégrés, que ce soit à l'intérieur ou à l'extérieur du châssis du téléviseur.

Le dispositif 30 comprend une enveloppe 31 en polymère présentant sensiblement une forme de cône tronqué. Dans une variante de ce mode de réalisation, l'enveloppe 31 peut être réalisée à partir d'une quelconque autre matière amorphe. L'enveloppe 31 est composée de six anneaux 32 placés l'un sur l'autre, de faible épaisseur et dont hauteur est beaucoup plus petite que leur diamètre, et est fermée par deux couvercles cylindriques 33 issus du même processus de fabrication. L'enveloppe 31 présente sensiblement une forme de cône tronqué. Le volume interne du dispositif 30 est rempli de nanopoudres 34 de carbone contenant des particules en forme de « bulbe ». Ces particules en forme de « bulbe permettent au dispositif 30 de générer son propre champ de torsion statique de forme droit de façon à augmenter la déviation des ondes de torsion incidentes.

Dans ce dispositif 30 se produit donc une conjugaison collaborative des effets de diffraction, par interférence des ondes de torsion gauche dans les passages de percolation existant à l'intérieur de la nanopoudre, et d'interaction avec le champ de torsion statique de forme droit généré par les particules en forme de bulbe.

La déviation du champ de torsion gauche incident produit par les composants électroniques du tube cathodique de l'écran est illustrée à la figure 3B.

On constate qu'en présence du dispositif 30 le champ de torsion gauche 12 est dévié vers le haut suivant un première portion de parcours 35 sensiblement équivalente à un demi-cercle, l'action du champ de torsion statique de forme droit généré par le dispositif permettant de repousser le champ de torsion gauche au delà d'un angle de déviation de 90°.

Le champ de torsion gauche du tube cathodique de l'écran, dont l'intensité a été amoindrie par le champ de torsion statique de forme droit interagit suivant une deuxième portion de parcours 36 avec le champ de torsion droit 13 généré par l'écran 11 de l'ordinateur. Cette interaction de deux champs 12 et 13 de tourbillons contraires conduit à leur atténuation jusqu'à un niveau négligeable sur une courte distance, sensiblement égale à un mètre.

Ainsi, l'utilisateur de l'ordinateur 11 est protégé des effets des champs de torsion qui peuvent causer des dommages aux cellules d'un organisme humain en donnant aux érythrocytes, encore appelés globules rouges, un mouvement tourbillonnaire et en entraînant l'inversion du sens de circulation des érythrocytes dans les vaisseaux sanguins (A.I. Veynik. Thermodynamique des processus réels. « Sciences et Techniques », 1991, p. 356).

### 6.4. Autres caractéristiques optionnelles et avantages

Les dispositifs de protection contre les effets des champs de torsion décrits ci-dessus sont destinés à être placés à l'intérieur ou contre les parois des téléphones cellulaires et/ou sur une paroi d'un écran de téléviseur ou d'ordinateur. Il est clair toutefois que ces dispositifs peuvent être aisément être adaptés à de nombreuses autres applications, comme, par exemple, les photocopieurs, sans sortir du cadre de l'invention.

Il est également clair que de nombreux autres modes de réalisation de l'invention peuvent être envisagés.

On peut ainsi prévoir, dans d'autres modes de réalisation de l'invention, des passages de dimensions diverses et adapter l'encombrement de l'élément de diffraction à la surface des circuits intégrés dont le rayonnement de torsion doit être intercepté.

Dans une variante du deuxième mode de réalisation particulier présenté ci-dessus, on peut également envisager de remplacer les particules de nanopoudre de carbone par des nanoparticules de silice, de métal ou encore de carbonate de calcium.

Dans encore d'autres modes de réalisation de l'invention, on peut aussi prévoir que:
- la grille comprend un maillage d'éléments courbes et/ou brisées entrecroisés ;
- des particules comprennent des bulbes présentant au moins une saillie prolongée par une pointe de façon à générer en outre un champ modifiant ladite direction du champ de torsion.

## Revendications

1. Dispositif de protection (20, 30) contre des effets d'un champ de torsion émis selon une direction par un appareil incluant des composants électroniques opérant dans une gamme de fréquence comprise entre un Mégahertz et trois Gigahertz comprenant un élément de diffraction (21), comprenant une pluralité de passages dont l'ouverture est de largeur inférieure ou égale à un millimètre dans au moins une direction, et au moins deux desdits passages présentant des pourtours de formes différentes, et un ensemble de particules (34) dont les intervalles séparateurs définissent ladite pluralité de passages, **caractérisé en ce que** lesdites particules sont réalisées dans une des matières appartenant au groupe comprenant :
- carbone ;
- silice ;
- métal ;
- carbonate de calcium.

2. Dispositif de protection selon la revendication 1, **caractérisé en ce que** ledit élément de diffraction (21) comprend une grille (22, 24), et **en ce que** la distribution desdits pourtours sur ladite grille est fractale.

3. Dispositif de protection selon la revendication 2, **caractérisé en ce qu'**au moins un desdits pourtours est de forme sensiblement circulaire ou elliptique.

4. Dispositif de protection selon l'une quelconque des revendications 2 et 3, **caractérisé en ce que** ladite grille (22, 24) comprend un maillage d'éléments courbes et/ou brisées entrecroisés.

5. Dispositif de protection selon l'une quelconque des revendications 2 à 4, **caractérisé en ce que** ladite grille (22,24) présente une densité supérieure ou égale à 19.

6. Dispositif de protection selon la revendication 1, **caractérisé en ce qu'**au moins une desdites particules comprend au moins un tube.

7. Dispositif de protection selon la revendication 6, **caractérisé en ce que** ledit bulbe comprend au moins une saillie prolongée par une pointe de façon à générer en outre un champ modifiant ladite direction du champ de torsion.

8. Dispositif de protection selon l'une des revendications 1 à 7, **caractérisé en ce que** ledit élément de diffraction (21) est enfermé dans une enveloppe fermée (31) en matériau amorphe.

9. Dispositif de protection selon la revendication 8, **caractérisé en ce que** la surface externe de ladite enveloppe fermée présente une surface externe définie par une pluralité de couches superposées (32) dans la direction dudit champ de torsion et formant des degrés successifs dont la largeur est supérieure à la hauteur.

## Claims

1. Device (20, 30) for protecting against effects of a torsion field emitted in one direction by an apparatus including electronic components operating in a frequency range of from one megahertz to three gigahertz, comprising a diffraction element (21) having a plurality of passages, the opening of which has a width of less than or equal to one millimetre in at least one direction, at least two of said passages having perimeters of different shapes, and a group of particles (34), the separating intervals of which define said plurality of passages, **characterised in that** said particles are made of one of the materials belonging to the group comprising:
- carbon;
- silica;
- metal;
- calcium carbonate.

2. Protecting device according to claim 1, **characterised in that** said diffraction element (21) comprises a grid (22, 24), and **in that** the distribution of said perimeters on said grid is fractal.

3. Protecting device according to claim 2, **characterised in that** at least one of said perimeters is substantially circular or elliptical in shape.

4. Protecting device according to either claim 2 or claim 3, **characterised in that** said grid (22, 24) comprises a meshing of interlaced curved and/or broken elements.

5. Protecting device according to any one of claims 2 to 4, **characterised in that** said grid (22, 24) has a density greater than or equal to 19.

6. Protecting device according to claim 1, **characterised in that** at least one of said particles comprises at least one bulb.

7. Protecting device according to claim 6, **characterised in that** said bulb comprises at least one projection prolonged by a point so as to further generate a field that modifies said direction of the torsion field.

8. Protecting device according to any one of claims 1 to 7, **characterised in that** said diffraction element (21) is enclosed in a closed casing (31) made of amorphous material.

9. Protecting device according to claim 8, **characterised in that** the outer surface of said closed casing has an outer surface defined by a plurality of superposed layers (32) in the direction of said torsion field and forming successive degrees, the width of which is greater than the height.

## Patentansprüche

1. Vorrichtung zum Schutz (20, 30) gegen Effekte eines von einem Gerät in einer Richtung ausgestrahlten Torsionsfelds, wobei das Gerät elektronische Komponenten enthält, die in einem Frequenzbereich zwischen einem Megahertz und drei Gigahertz arbeiten, wobei die Schutzvorrichtung ein Beugungselement (21) aufweist, das eine Mehrzahl Durchführungen umfasst, deren Öffnung eine Breite kleiner gleich einem Millimeter in mindestens einer Richtung hat und wobei mindestens zwei der Durchführungen Außenlinien unterschiedlicher Formen aufweisen, und eine Menge von Teilchen (34) aufweist, deren Trennabstände die Mehrzahl der Durchführungen definieren, **dadurch gekennzeichnet, dass** die Teilchen aus einem der Materialien hergestellt sind aus der Gruppe umfassend Folgendes:
- Kohlenstoff;
- Siliciumdioxid;
- Metall;
- Calciumcarbonat.

2. Schutzvorrichtung nach Anspruch 1, **dadurch gekennzeichnet, dass** das Beugungselement (21) ein Gitter (22, 24) umfasst, und dass die Verteilung der Außenlinien auf dem Gitter fraktal ist.

3. Schutzvorrichtung nach Anspruch 2, **dadurch gekennzeichnet, dass** mindestens eine der Außenlinien eine im Wesentlichen kreisförmige oder elliptische Form hat.

4. Schutzvorrichtung nach einem der Ansprüche 2 und 3, **dadurch gekennzeichnet, dass** das Gitter (22, 24) eine Vernetzung von gitterartig gebogenen und/oder gebrochenen Elementen umfasst.

5. Schutzvorrichtung nach einem der Ansprüche 2 bis 4, **dadurch gekennzeichnet, dass** das Gitter (22, 24) eine Dichte größer gleich 19 aufweist.

6. Schutzvorrichtung nach Anspruch 1, **dadurch gekennzeichnet, dass** mindestens eines der Teilchen mindestens eine Knolle umfasst.

7. Schutzvorrichtung nach Anspruch 6, **dadurch gekennzeichnet, dass** die Knolle mindestens einen durch eine Spitze verlängerten Vorsprung umfasst, um außerdem ein Feld zu erzeugen, das die Richtung des Torsionsfelds ändert.

8. Schutzvorrichtung nach einem der Ansprüche 1 bis 7, **dadurch gekennzeichnet, dass** das Beugungselement (21) in eine geschlossene Umhüllung (31) aus amorphem Material eingeschlossen ist.

9. Schutzvorrichtung nach Anspruch 8, **dadurch gekennzeichnet, dass** die Außenfläche der geschlossenen Umhüllung eine Außenfläche aufweist, die von mehreren in der Richtung des Torsionsfelds übereinander liegenden Schichten (32) definiert wird, die aufeinanderfolgende Abstufungen bilden, deren Breite größer als die Höhe ist.
